(19) Europäisches Patentamt
European Patent Office
Office européen des brevets

(11) Veröffentlichungsnummer: **0 095 101**
**B1**

(12) # EUROPÄISCHE PATENTSCHRIFT

(45) Veröffentlichungstag der Patentschrift: **08.01.86**

(21) Anmeldenummer: **83104696.6**

(22) Anmeldetag: **13.05.83**

(51) Int. Cl.[4]: **C 07 C 43/32,** C 07 C 43/303, C 07 C 41/60, C 07 C 41/50 // C07C29/128

(54) **Verfahren zur Herstellung von aliphatischen Alkoxyverbindungen.**

(30) Priorität: **25.05.82 DE 3219541**
**20.01.83 DE 3301719**

(43) Veröffentlichungstag der Anmeldung:
**30.11.83 Patentblatt 83/48**

(45) Bekanntmachung des Hinweises auf die Patenterteilung:
**08.01.86 Patentblatt 86/2**

(84) Benannte Vertragsstaaten:
**BE CH DE FR GB LI NL**

(56) Entgegenhaltungen:
**THE JOURNAL OF ORGANIC CHEMISTRY, Band 25, Nr. 10, 13. Oktober 1960, Seiten 1814-1815, American Chemical Society, USA, M. STILES et al.: "A convenient general method for the preparation of aldehydes"**
**JOURNAL OF HETEROCYCLIC CHEMISTRY, Band 13, Nr. 5, Oktober 1976, Seiten 1125-1128, Hetero Corporation, USA, J. SOULIER et al.: "Synthèse de quelques alcoxy-2 et aryloxy-2 dioxolannes-1,3"**

**Die Akte enthält technische Angaben, die nach dem Eingang der Anmeldung eingereicht wurden und die *nicht in dieser Patentschrift enthalten sind.***

(73) Patentinhaber: **BASF Aktiengesellschaft, Carl-Bosch-Strasse 38, D-6700 Ludwigshafen (DE)**

(72) Erfinder: **Eckhardt, Heinz, Dr., Hessheimer Strasse 50, D-6710 Frankenthal (DE)**
Erfinder: **Halbritter, Klaus, Dr., Schwarzwaldstrasse 19, D-6800 Mannheim 1 (DE)**

**EP 0 095 101 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft ein Verfahren zur Herstellung von aliphatischen Alkoxyverbindungen durch Umsetzung von 2-substituierten (1,3)-Dioxolanen mit aliphatischen Alkoholen bei einer Temperatur von 0 bis 150° C in Gegenwart eines Katalysators.

Trialkylorthoformiate kann man durch Umsetzen von Chloroform mit Metallalkoholaten oder durch Umsetzen von Blausäure mit Alkoholen herstellen (Houben-Weyl, Methoden der Organischen Chemie, Band VI/3, Seiten 300, 306).

Diese Verfahren haben den Nachteil, dass ein grosser Salzanfall in Kauf genommen werden muss und korrosionsfeste Apparaturen notwendig sind. Weitere Nachteile sind die Toxizität der Einsatzstoffe; die MAK-Werte sind für $CHCl_3$: 50 und für HCN: 10 ppm (Ullmanns Encyklopädie der technischen Chemie, Band 2/2, Seite 620) und ergeben damit Schwierigkeiten bezüglich Betriebsicherheit und Umweltschutz. Soulier, M. Farines, R.M. Authier und M. Fournier (J. Heterocycl. Chem., Band 13, Seiten 1125 bis 1128 (1976)) beschreiben die Umsetzung von Alkylenglykolen mit Trialkylorthoformiaten bei Temperaturen von 100 bis 150° C, auch in Gegenwart von Säuren als Katalysatoren, zu 2-Alkoxy-(1,3)-dioxolanen. Zur Verschiebung des Gleichgewichts wird der gebildete Alkohol während der Reaktion ständig abdestilliert. Ausbeuten von 13 bis 74%, z.B. bei der Herstellung (ohne Katalysator) von 2-Methoxy-1,3-dioxolan 26% und der 2-Ethoxy-Verbindung (mit Katalysator) 43%, werden erhalten. Es wird ausdrücklich festgestellt, dass die Umsetzung vorteilhaft in Abwesenheit von Katalysatoren durchgeführt wird.

Es ist bekannt (Lorette et al., J. Org. Chem., 1959, Seiten 1731 bis 1733), 2,2-Dimethoxypropan durch Ketalisierung von Ketonen mit Alkanolen in Gegenwart saurer Ionenaustauscher und bei tiefer Temperatur herzustellen. Da das Gleichgewicht dieser Reaktion jedoch weitgehend auf der Seite der Ausgangsstoffe (Umsatz mit Aceton und Methanol bei 24° C: 11%) liegt, lehrt Lorette, den Umsatz durch eine Reaktion bei sehr tiefen Temperaturen (Umsatz mit Aceton und Methanol bei −28° C: 32%) zu verbessern. Diese Verfahrensweise ist jedoch wegen der energieintensiven notwendigen Kältetechnik und wegen der bei den tiefen Temperaturen geringen Reaktionsgeschwindigkeiten unwirtschaftlich und eignet sich ausserdem wegen der Bildung verschiedener Azeotrope schlecht für die kontinuierliche Gewinnung des Ketals aus den entstehenden Reaktionsgemischen.

Auch das Verfahren der DE-OS 26 36 278, die Umsetzung bei 15 bis 80° C in Anwesenheit von Mineralsäuren und von äquivalenten Mengen (bezogen auf das Keton) wasserfreiem Calciumsulfat durchzuführen, ist wegen des hohen Verbrauchs an Calciumsulfat und der Regenerierungskosten des anfallenden Hydrats nachteilig. Ausserdem werden aromatische, aliphatische oder cycloaliphatische Kohlenwasserstoffe als Lösungsmittel verwendet, wie alle Beispiele zeigen.

In der deutschen Offenlegungsschrift 29 29 827 wird die Umsetzung von Aceton mit Methanol in Gegenwart saurer Ionenaustauscher mit einem Acetonüberschuss (Molverhältnis 3,6 bis 4,4 zu 1 Methanol) bei 15 bis 30° C und anschliessende Trennung des Reaktionsgemischs in einer 40- bis 60bödigen Destillationskolonne beschrieben. Als wesentlicher Nachteil ist anzusehen, dass die gesamte Menge an nicht umgesetztem Aceton und Methanol destilliert werden muss, was etwa der 25fachen Menge des Wertproduktes entspricht.

Es wurde nun gefunden, dass man aliphatische Alkoxyverbindungen der Formel

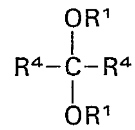
(I)

worin ein Rest $R^4$ ein Wasserstoffatom und der andere Rest $R^4$ die Gruppe $-OR^1$ bezeichnen oder beide Reste $R^4$ jeweils für einen Methylrest stehen, die einzelnen Reste $R^1$ gleich oder verschieden sein können und jeweils einen aliphatischen Rest mit 1 bis 6 Kohlenstoffatomen bedeuten, vorteilhaft erhält, wenn man 2-substituierte (1,3)-Dioxolane der Formel

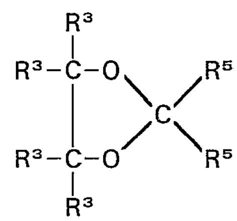
(II)

worin ein Rest $R^5$ ein Wasserstoffatom und der andere Rest $R^5$ den Rest

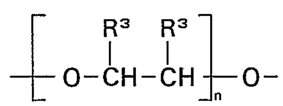

bedeuten oder beide Reste $R^5$ jeweils für einen Methylrest stehen, die einzelnen Reste $R^2$ und $R^3$ gleich oder verschieden sind und jeweils einen aliphatischen Rest bedeuten, $R^3$ auch für ein Wasserstoffatom, einen cycloaliphatischen, araliphatischen oder aromatischen Rest steht, und n eine ganze Zahl von 0 bis 3 bezeichnet, mit aliphatischen Alkoholen der Formel

$$R^1-OH \qquad (III)$$

worin $R^1$ die vorgenannte Bedeutung besitzt, bei einer Temperatur von 0 bis 150° C

a) in Gegenwart eines Halogenids von Elementen der Gruppen IIb, IIIa, IVb, Va des Periodischen Systems und/oder von Eisen, Zinn, Silicium als Katalysatoren und gegebenenfalls einer Säure oder auch

b) wenn ein Rest $R^5$ ein Wasserstoffatom und der andere Rest $R^5$ den Rest

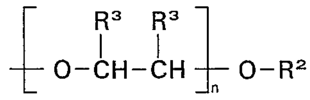

bedeuten, in Gegenwart einer Säure umsetzt.

Die Umsetzung kann für den Fall der Verwendung von Methanol und 2-Ethoxy-4-methyl-(1,3)-dioxolan oder 2,2-Dimethyl-(1,3)-dioxolan durch die folgenden Formeln wiedergegeben werden:

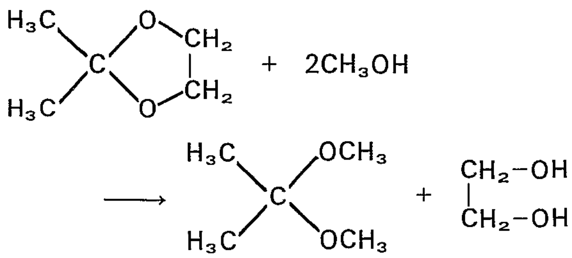

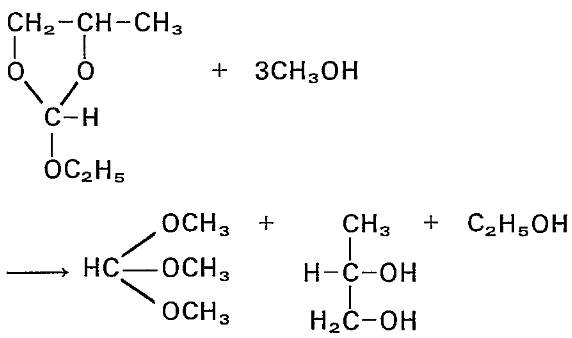

Im Vergleich zu den bekannten Verfahren liefert das Verfahren nach der Erfindung auf einfacherem und wirtschaftlicherem Wege aliphatische Alkoxyverbindungen in Gestalt von 2,2-Dialkoxypropanen und Trialkylorthoformiaten in hoher Ausbeute und Reinheit. Eine Umsetzung bei Temperaturen unter 0° C und ein Verbrauch hoher Mengen an Hilfsstoffen werden vermieden. Auch das bei der Reaktion gebildete Alkylenglykol wird in hoher Ausbeute und Reinheit erhalten. Weitere Vorteile sind die geringe Toxizität der Edukte, ein sehr geringer Salzanfall (aus dem Katalysator) und nur flüssige organische Nebenprodukte.

Alle diese vorteilhaften Ergebnisse des erfindungsgemässen Verfahrens sind im Hinblick auf den Stand der Technik überraschend. Im Hinblick auf die Lehre der Veröffentlichung in J. Heterocyclic Chem. (loc. cit.) hätte man bei den erfindungsgemässen niedrigeren Temperaturen keine Spaltung des 2-Alkoxy-1,3-dioxolans erwartet. Angesichts der Lehre der Veröffentlichung in J. Org. Chem. (loc. cit.) hätte man bei den erfindungsgemässen höheren Temperaturen keine oder zumindest nur geringe Bildung der 2,2-Dialkoxypropane vermutet; auch ist es überraschend, dass die erfindungsgemässen Katalysatoren gerade zur Herstellung von 2,2-Dialkoxypropanen und Trialkylorthoformiaten und damit zur Spaltung des Dioxolans eine wesentliche Rolle spielen.

Als 2-substituierte (1,3)-Dioxolane kommen aliphatische 2-Alkoxy- und 2,2-Dimethyl-(1,3)-dioxolane in Frage. Die als Ausgangsprodukte benötigten 2-Alkoxy-(1,3)-dioxolane können nach bekannten Verfahren, z.B. (Houben-Weyl, Methoden der Organischen Chemie, Band VI/3, Seite 311) durch Umsetzung von Ethylenoxid oder Epichlorhydrin mit Ameisensäureethylester in Gegenwart von Bortrifluorid oder (Chem. Ber. *91*, 650 bis 653 (1958)) durch Umsetzung von Orthoameisensäuretriethylester und Propylenglykol in Gegenwart von Schwefelsäure erhalten werden. Ebenfalls kann man 2-Alkoxy-(1,3)-dioxolane der Formel

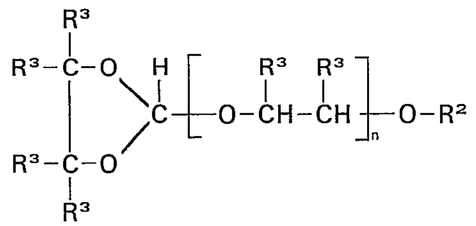 (IIa)

worin $R^2$ und $R^3$ gleich oder verschieden sind und jeweils einen aliphatischen Rest bedeuten, $R^3$ auch für ein Wasserstoffatom, einen cycloaliphatischen, araliphatischen oder aromatischen Rest steht, und n eine ganze Zahl von 0 bis 3 bezeichnet, durch Umsetzung von Ameisensäurealkylestern der Formel

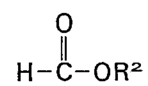 (IV)

in der $R^2$ die vorgenannte Bedeutung besitzt, mit Oxiranen der Formel

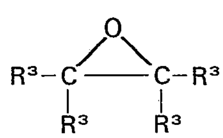 (V)

in der $R^3$ die vorgenannte Bedeutung besitzt, in Gegenwart von Borhalogeniden oder Antimon-V-halogeniden erhalten. Bevorzugt sind zur Herstellung der Ausgangsstoffe IIa Molverhältnisse von 0,001 bis 1 Ausgangsstoff V zu Ausgangsstoff IV, und Temperaturen von −80 bis +200° C, zweckmässig von 0 bis 100° C, vorteilhaft von 0 bis 50° C. Bevorzugte Katalysatoren zur Herstellung der Ausgangsstoffe IIa sind Antimon-V-bromid, Antimon-V-fluorid und vorteilhaft Antimon-V-chlorid, Bortribromid, Bortrichlorid oder insbesondere Bortrifluorid. Die Konzentration des Katalysators im Reaktionsgemisch zur Herstellung der Ausgangsstoffe II kann zwischen 0,001 und 40 Gew.-% betragen, bevorzugt werden 0,05 bis 0,15 Gew.-% eingesetzt.

Die als Ausgangsstoffe II benötigten 2,2-Dimethyl-(1,3)-dioxolane können nach bekannten Verfahren, z.B. der in DE-PS 1 086 241 beschriebenen Umsetzung von Aceton mit Ethylenoxid oder Epichlorhydrin in Gegenwart von säureaktivierten Bentoniten, erhalten werden.

Man kann die Ausgangsstoffe II und III in stöchiometrischer Menge oder im Überschuss jeder Komponente zur anderen, zweckmässig in einem Verhältnis von 2 bis 100, bevorzugt 4 bis 100, vorteilhaft von 4 bis 40 Mol Ausgangsstoff III je Mol Ausgangsstoff II umsetzen. Bevorzugte Ausgangsstoffe II, III, Stoffe IV, V und dementsprechend bevorzugte Endstoffe I sind solche, in deren Formeln ein Rest $R^4$ ein Wasserstoffatom und der andere Rest $R^4$ die Gruppe $-OR^1$ bezeichnen oder beide Reste $R^4$ jeweils für einen Methylrest stehen, die einzelnen Reste $R^1$ gleich oder verschieden sind und jeweils einen Alkylrest mit 1 bis 6, vorteilhaft 1 bis 4 Kohlenstoffatomen bedeuten, ein Rest $R^5$ ein Wasserstoffatom und der andere Rest $R^5$ den Rest

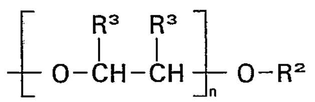

bedeuten, oder beide Reste $R^5$ jeweils für einen Methylrest stehen, die einzelnen Reste $R^2$ und $R^3$ gleich oder verschieden sind und jeweils einen Alkylrest mit 1 bis 4 Kohlenstoffatomen bedeuten, $R^3$ auch für ein Wasserstoffatom, einen durch Halogenatome, zweckmässig Bromatome oder insbesondere Chloratome, vorteilhaft 1 Halogenatom, und/oder Alkoxygruppen, vorteilhaft 1 Alkoxygruppe, zweckmässig mit 1 bis 4 Kohlenstoffatomen, substituierten Alkylrest mit 1 bis 3 Kohlenstoffatomen, einen Cycloalkylrest mit 5 bis 8 Kohlenstoffatomen, einen Aralkylrest oder Alkylarylrest mit 7 bis 12 Kohlenstoffatomen oder einen Phenylrest, wobei die Phenylgruppe in den vorgenannten 3 Resten durch Alkoxygruppen, Alkylgruppen mit jeweils 1 bis 4 Kohlenstoffatomen, und/oder Halogenatome, vorteilhaft Brom- und insbesondere Chloratome, substituiert sein kann, steht, und n eine ganze Zahl von 0 bis 3, zweckmässig 0 bis 2, bevorzugt 0 oder 1, insbesondere 0, bezeichnet. Als Ausgangsstoff II kann man auch ein Gemisch der 2-Alkoxy-(1,3)-dioxolane, z.B. mit 66 bis 81 Gew.% 2-Alkoxy-(1,3)-dioxolanen mit n = 0, mit 4 bis 13 Gew.% 2-Alkoxy-(1,3)-dioxolanen mit n = 1, mit 1 bis 3,3 Gew.% Dioxolanen mit n = 2, mit 0,2 bis 1 Gew.% Dioxolanen mit n = 3, bezogen auf die Gewichtsmenge des Gesamtreaktionsgemisches, wie es bei den vorgenannten Herstellungsverfahren der Dioxolane II entstehen kann, verwenden.

So kommen beispielsweise folgende 2-Alkoxy-(1,3)-dioxolane II in Betracht: 2-Methoxy-, 2-Ethoxy-, 2-Propoxy-, 2-Isopropoxy-, 2-Butoxy-, 2-Isobutoxy-, 2-sek.-Butoxy-, 2-Pentoxy-, 2-Isopentoxy-, 2-(2'-Methoxy)-ethoxy-, 2-(2'-Ethoxy)-ethoxy-, 2-(2'-Methoxy)-propoxy-, 2-(2'-Ethoxy)-propoxy-(1,3)-dioxolan; vorgenannte, in 4- und 5-Stellung gleich oder verschieden durch 1, 2, 3 oder 4 Methyl-, Ethyl-, n-Propyl-, i-Propyl-, n-Butyl-, i-Butyl-, s-Butyl-, Chlormethyl-, 1'-Chlorethyl-, 2'-Chlorethylgruppen substituierte Dioxolane; bevorzugt sind 2-Methoxy- und 2-Ethoxy-1,3-dioxolane, die in 4- und 5-Stellung 2, zweckmässig 3 Wasserstoffatome und 2, zweckmässig 1 Methyl- und/oder Chlormethylgruppen tragen, oder vorteilhaft unsubstituiert sind.

So kommen beispielsweise folgende 2,2-Dimethyl-(1,3)-dioxolane II in Betracht: 2,2-Dimethyl-(1,3)-dioxolan, in 4 und/oder 5-Stellung gleich oder verschieden durch 1, 2, 3 oder 4 Methyl-, Ethyl-, n-Propyl-, i-Propyl-, n-Butyl-, i-Butyl-, s-Butyl-, Chlormethyl-, Methoxymethyl-, Cyclohexyl-, Tolyl-, Benzyl-, Phenylgruppen substituierte 2,2-Dimethyl-(1,3)-dioxolane; bevorzugt sind 2,2-Dimethyl- und 2,2,4-Trimethyl-(1,3)-dioxolan.

Folgende Ausgangsstoffe III sind z.B. geeignet: Methyl-, Ethyl-, Propyl-, Isopropyl-, Butyl-, Isobutyl-, sek.-Butylalkohol; bevorzugt sind Methanol oder Ethanol.

Die Umsetzung wird bei einer Temperatur von 0 bis 150° C, im allgemeinen von 0 bis 100° C, zweckmässig zwischen 0 und 100° C, vorzugsweise von 20 bis unterhalb 100° C, vorteilhaft von 20 bis 90° C, insbesondere vorteilhaft von 30 bis 80° C, bevorzugt 35 bis 80° C, insbesondere 35 bis 75° C, insbesondere 40 bis 75° C, bei vermindertem Druck, erhöhtem Druck oder bevorzugt drucklos, diskontinuierlich oder bevorzugt kontinuierlich durchgeführt.

Bevorzugt arbeitet man in einem Temperaturbereich, der zwischen 0° C und dem Siedepunkt des Reaktionsgemisches liegt. Als Lösungsmittel können aliphatische oder aromatische Kohlenwasserstoffe, z.B. Hexan, Benzol, Toluol, Xylole verwendet werden; bevorzugt führt man die Reaktion in überschüssigem Ausgangsstoff III durch. Die Lösungsmittelmenge bzw. der als Lösungsmittel dienende, über vorgenannten Mengen Ausgangsstoff III, bezogen auf Ausgangsstoff II, liegende Überschuss an Ausgangsstoff III beträgt zweckmässig zwischen 0 und 100 Gew.% Lösungsmittel (Ausgangsstoff III), bezogen auf Ausgangsstoff II.

Als Katalysatoren werden Säuren und/oder Halogenide verwendet. Die Umsetzung wird vorteilhaft mit einer Menge oberhalb 0,000005, bevorzugt mit einer Menge von 0,00005 bis 1 Äquivalenten Säure; im Falle von Alkancarbonsäuren mindestens 0,05, bevorzugt 0,05 bis 1, insbesondere 0,08 bis 0,5; im Falle von Halogenalkancarbonsäuren und organischen Sulfonsäuren oberhalb 0,000005, bevorzugt 0,00005 bis 0,1, insbesondere 0,0005 bis 0,01; im Falle von anorganischen Säuren oberhalb 0,00005, bevorzugt zwischen 0,00005 und 0,2, insbesondere von 0,0005 bis 0,01 Äquivalenten Säure, bezogen auf 1 Mol Ausgangsstoff II, durchgeführt. Es werden anorganische oder organische Säuren mit einem $pK_s$-Wert kleiner als 6, bevorzugt kleiner als 5, verwendet. Anstelle einbasischer Säuren können auch äquivalente Mengen mehrbasischer Säuren zur Anwendung gelangen. Beispielsweise sind folgende Säuren geeignet: Chlorwasserstoff, Bromwasserstoff, Jodwasserstoff, Schwefelsäure, Phosphorsäure, Salpetersäure, 12-Molybdophosphorsäure, 12-Molybdokieselsäure, 12-Wolframphosphorsäure, 12-Wolframkieselsäure, 12-Wolframborsäure; Sulfonsäuren wie Benzol-und p-Toluolsulfonsäure, Methanolsulfonsäure, Trifluormethansulfonsäure; Bor enthaltende Säuren wie Borsäure, Borfluorwasserstoffsäure; aliphatische Carbonsäuren wie Chlor- oder Fluoressigsäure, Dichloressigsäure, Difluoressigsäure, Trifluoressigsäure, Perfluorpropionsäure, Trichloressigsäure, Oxalsäure, Ameisensäure, Essigsäure, Propionsäure, Buttersäure, Isobuttersäure, Glykolsäure, Milchsäure, Brenztraubensäure, Weinsäure, Zitronensäure, β-Oxybuttersäure, Caprylsäure, Trimethylessigsäure, α- bzw. β-Chlorpropionsäure, Bernsteinsäure, Isovaleriansäure, Valeriansäure, Glutarsäure, Adipinsäure; cycloaliphatische, araliphatische, aromatische Carbonsäuren wie Benzoesäure, 2,3-, 2,4-, 2,5-, 2,6-Dimethylbenzoesäure, o-, m- bzw. p-Oxybenzoesäure, Mellithsäure, Phenylpropionsäure, o-, m- bzw. p-Chlorbenzoesäure, Cyclohexancarbonsäure, Cyclopentancarbonsäure, Phenylessigsäure, α- bzw. β-Naphthoesäure, Phthalsäure, o-, m- bzw. p-Toluylsäure, o-, m- bzw. p-Nitrobenzoesäure, Isophthalsäure, Terephthalsäure; saure Ionenaustauscher; Montmorillonite, z.B. der Typ KSF oder K 10, oder entsprechende Gemische. Die Säuren können in verdünnter oder konzentrierter Form, im Gemisch miteinander und/oder mit einem Lösungsmittel, insbesondere dem Ausgangsstoff III, angewendet werden. Bei verdünnten, alkoholischen Säuren sind 1- bis 20-gew.%ige Säuren, z.B. 1- bis 10-gew.%ige Salzsäure, 1- bis 10-gew.%ige Schwefelsäure oder 1- bis 10-gew.%ige Phosphorsäure, vorteilhaft. Bevorzugt sind Salzsäure, Schwefelsäure, Phosphorsäure, 12-Wolframkieselsäure, Salpetersäure, Benzolsulfonsäure, p-Toluolsulfonsäure, Oxalsäure, Ameisensäure, Essigsäure, Trifluoressigsäure, Propionsäure, Trifluormethansulfonsäure, Monochloressigsäure, Di-, Trichloressigsäure. Der pH-Wert der Umsetzung beträgt zwischen 0 und 7, vorzugsweise zwischen 0 und 6, insbesondere von 2 bis 5.

Die Umsetzung wird ebenfalls in Gegenwart von sauren Ionenaustauschern bzw. Kationenaustauschern, vorzugsweise sauren Kunstharzaustauschern, durchgeführt. Solche Austauscher sind z.B. alle in Houben-Weyl, Methoden der Organischen Chemie, Band I/1, Seite 528, Tabelle 3 beschriebenen Kationenaustauscher. Vorzugsweise verwendet man stark und mittelstark saure Austauscher, z.B. Phenol- oder Polystyrolsulfonsäureharze, Styrolphosphonsäure-, Styrolphosphinsäureharze, Resorcinharze, aliphatische oder aromatische Carbonsäureharze oder entsprechende saure Harze enthaltende Austauscher, z.B. bifunktionelle Kondensationsharze. So kommen z.B. vorgenannte Harze, die im Handel unter der Bezeichnung ®Lewatit S 100, ®Lewatit SP 120, ®Lewasorb, ®Dowex 50 W × 8, ®Amberlyst 15 erhältlich sind, in Betracht. Die Menge der verwendeten Ausgangsstoffe bzw. des Austauschers hängt von der Selektivität bzw. der Zahl der austauschaktiven Gruppen der verwendeten Austauscher bei der Reaktionstemperatur ab, im allgemeinen verwendet man 1 bis 20 Gew.% Austauscher, bezogen auf Ausgangsstoff II, im diskontinuierlichen Betrieb und im kontinuierlichen Betrieb 10 bis 100 Gew.%, vorzugsweise 20 bis 70 Gew.% Austauscher, bezogen auf die durchschnittlich pro Stunde durch den Austauscher geleitete Menge an Ausgangsstoff II. Die im trockenen Austauscher enthaltene Wassermenge kann je nach Struktur bis zu 50 Gew.% betragen. Zweckmässigerweise wird das Wasser vor Verwendung des Austauschers mit einer entsprechenden Menge von Ausgangsstoff II ausgewaschen. Form und Korngrösse des Austauschers können in einem weiten Bereich beliebig gewählt werden. Bezüglich Herstellung und Einzelheiten der Verwendung von Ioneaustauschern wird auf das Kapitel „Ionenaustauscher" des genannten Werkes verwiesen.

Als Katalysatoren werden auch vorgenannte Halogenide, im allgemeinen die als Lewis-Säuren reagierenden Halogenide, zweckmässig in einer Menge oberhalb 0,00001, bevorzugt von 0,0001 bis 0,1, insbesondere 0,001 bis 0,01 Mol Halogenid, bezogen auf 1 Mol Ausgangsstoff II, verwendet. Vorteilhafte Katalysatoren sind die Fluoride, insbesondere die Chloride von Al, Sb, B, Fe, As, P, Hg, Sn, Zn, Zr, Si und Ti, bevorzugt $BF_3$, $AlCl_3$, $FeCl_3$, $SbF_5$, $SbCl_5$, $SnCl_4$ und $ZnCl_2$. Die Anordnung des Periodischen Systems entspricht D'Ans-Lax, Taschenbuch für Chemiker und Physiker (Springer, Berlin, 1967), Band 1, Seite 63, entsprechend Weast, Handbook of Chemistry and Physics (Chemical Rubber Co., Cleveland, 50. ed.), Seite B 3.

Die Reaktion kann wie folgt durchgeführt werden: ein Gemisch von Katalysator, Ausgangsstoff II und Ausgangsstoff III und gegebenenfalls von Lösungsmitteln wird während 0,1 bis 6 Stunden bei der Reaktionstemperatur, zweckmässig der Siedetemperatur des Reaktionsgemischs, gehalten. Nach Ende der Reaktion wird vorteilhaft der Katalysator mit Basen neutralisiert und der Endstoff in üblicher Verfahrensweise, z.B. durch Destillation, isoliert. Die Neutralisation des Katalysators ist nicht notwendig, wenn sich Endstoff I und Alkohol III aufgrund ihres Siedeverhaltens gleichzeitig als Gemisch abdestillieren lassen, z.B. Methanol und Trimethylorthoformiat oder Methanol und 2,2-Dimethoxypropan. Zweckmässig wird während der Reaktion über eine Kolonne ständig ein Gemisch aus Endstoff, z.B. 2,2-Dimethoxypropan, und Alkanol III, z.B. Methanol, abdestilliert. Bei Verwendung eines Ionenaustauschers wird in einer zweckmässigen Verfahrensweise ein Gemisch aus Ausgangsstoffen II und III durch eine Säule gepumpt, die mit dem Ionenaustauscher gefüllt ist. Eine bevorzugte Verfahrensweise ist die Durchführung der Reaktion in einer Reaktionskolonne, z.B. einer Glockenbodenkolonne, wobei neben der Reaktion gleichzeitig eine Auftrennung des Reaktionsgemisches erfolgt, so dass ein vollständiger Umsatz an Ausgangsstoff II sowohl bei einer diskontinuierlichen als auch bei einer kontinuierlichen Fahrweise möglich ist. Obwohl dabei jede der oben genannten Katalysatorsäuren einsetzbar ist, sind einerseits unlösliche Katalysatoren wie Ionenaustauscher oder andererseits flüchtige Säuren mit einem Siedepunkt zwischen dem des Alkohols III und dem Endstoff I, im Falle der Herstellung von 2,2-Dialkoxypropanen I mit einem Siedepunkt zwischen 60 und 120° C, besonders geeignet. Die Reaktionskolonne wird vorzugsweise so betrieben, dass der Ausgangsstoff II im oberen oder mittleren Drittel der Kolonne und der Alkohol III von unten zugeführt wird. Zweckmässig wird der Ausgangsstoff II 5 bis 50, bevorzugt 10 bis 30 theoretische Böden vom oberen Kolonnenende entfernt zugeführt. Der Endstoff I wird zusammen mit dem nicht umgesetzten Alkohol III am Kolonnenkopf entnommen, während das Diol am unteren Ende der Kolonne anfällt. Das Kopfdestillat kann durch einfache Destillation, im Falle der Herstellung von 2,2-Dialkoxypropanen I durch Azeotrop-Destillation mit einem Kohlenwasserstoff, getrennt werden, wobei der Endstoff in hoher Reinheit anfällt; für einige Verwendungsmöglichkeiten, z.B. Veresterung von Carbonsäuren, Ketalisierung höherer Ketone, ist diese Auftrennung des Destillats nicht notwendig. Die Reinigung des Diols kann durch Destillation oder Extraktion mit einem Alkan oder Alkangemisch erfolgen.

An die Reinheit der benötigten 2-substituierten (1,3)-Dioxolane II sind keine besonderen Anforderungen zu stellen; zur Durchführung des erfindungsgemässen Verfahrens ist es meist nicht notwendig, die nach den obengenannten Verfahren hergestellten 2-substituierten (1,3)-Dioxolane II zu isolieren. Man kann das Endgemisch der Herstellung des Ausgangsstoffs II, das das Hauptprodukt Ausgangsstoff II, den Katalysator, Nebenprodukte und gegebenenfalls Lösungsmittel enthält, im Falle der Herstellung von Trialkylorthoformiaten I direkt und gegebenenfalls ohne weitere Katalysatorzugabe, im Falle der Herstellung von 2,2-Dialkoxypropanen I direkt nach Abtrennung des Katalysators und des noch vorhandenen Acetons, für die erfindungsgemässe Reaktion verwenden.

Die nach dem Verfahren der Erfindung herstellbaren 2,2-Dialkoxypropane und Orthoformiate sind wertvolle Ausgangsstoffe für die Herstellung von Insektiziden, Pflanzenschutzmitteln und Pharmazeutika. Ebenfalls dienen sie als wasserbindende Mittel in chemischen Synthesen. Die 2,2-Dialkoxypropane dienen auch zur Herstellung von Acetonketalen höherer Alkohole, indem man z.B. das Dimethylketal mit diesen Alkoholen umketalisiert. Der letztgenannte Weg ist in aller Regel einfacher als die direkte Ketalisierung des Acetons mit den höheren Alkoholen. Die Orthoformiate können z.B. als $C_1$-Bausteine in der Synthese von Heterocyclen, Wirkstoffen im Pharma- und Pflanzenschutzbereich, von Farbstoffen (Cyanine, Triarylmethane) und als Schutzmittel bestimmter funktioneller Gruppen verwendet werden. Bezüglich der Verwendung wird auf die vorgenannte Literatur und Ullmanns Encyklopädie der technischen Chemie, Band 13, Seite 275, sowie Band 3, Seiten 13 bis 17, verwiesen.

*Beispiel 1*

Eine Mischung von 900 Gramm Methanol und 300 Gramm 2-Methoxy-4-methyl-(1,3)-dioxolan wurde bei 60° C durch eine 30 cm lange, mit 30 Gramm unter dem Namen ®Lewatit SP 120 im Handel erhältlichen Ionenaustauscher gefüllte Säule gepumpt. Die Verweilzeit beträgt 30 Minuten. Man erhält (gaschromatographisch geprüft) 119 Gramm (45% der Theorie) Trimethylorthoformiat vom Kp. 102° C und 158 Gramm nicht umgesetztes 2-Methoxy-4-methyl-(1,3)-dioxolan.

*Beispiele 2 bis 18*

Eine Mischung aus 60 Gramm Methanol und 20 Gramm 2-Methoxy-4-methyl-(1,3)-dioxolan wurde bei 65 bis 70° C mit der in Tabelle 1 angegegenen Menge Katalysator während der angegebenen Reaktionszeit mit den angegebenen Ausbeuten umgesetzt.

*Beispiele 19 bis 21*

Eine Mischung aus 60 Gramm des in Tabelle 2 angegebenen Alkohols und 20 Gramm 2-Methoxy-4-methyl-(1,3)-dioxolan wurde bei 65° C mit 0,2 Gramm Trifluoressigsäure während der angegebenen Reaktionszeit mit den angegebenen Ausbeuten an Trialkylorthoformiat umgesetzt. Im Falle der Beispiele 20 und 21 wird das nicht zu Orthoformiat umgesetzte 2-Methoxy-4-methyl-(1,3)-dioxolan im wesentlichen zu 2-Ethoxy- bzw. 2-Butoxy-4-methyl-(1,3)-dioxolan umgewandelt.

*Tabelle 1*

| Beispiel | Katalysator | Gramm | Zeit in Minuten | Ausbeute in % der Theorie | Selektivität in % der Theorie, bezogen auf umgesetzten Ausgangsstoff II |
|---|---|---|---|---|---|
| 2 (Vergleich) | $CH_3COOH$ | 0,1 | 60 | 0 | — |
| 3 | $CH_3COOH$ | 1 | 60 | 33,5 | 94,6 |
| 4 | $CH_3COOH$ | 10 | 20 | 31,5 | 92,2 |
| 5 | $C_2H_5COOH$ | 5 | 60 | 43,9 | 92,8 |
| 6 | $CF_3COOH$ | 0,01 | 10 | 42,1 | 95,3 |
| 7 | $CF_3COOH$ | 1 | 10 | 45,6 | 95,7 |
| 8 | $CF_3SO_3H$ | 0,1 | 2 | 46,7 | 94,9 |
| 9 | $CF_3SO_3H$ | 1 | 10 | 45,6 | 95,2 |
| 10 | p-Toluolsulfonsäure | 1 | 10 | 42,3 | 95,5 |
| 11 | $H_3PO_4$ | 0,2 | 30 | 41,4 | 94,5 |
| 12 | $H_2SO_4$ | 0,2 | 10 | 44,9 | 95,1 |

| Beispiel | Katalysator | Gramm | Zeit in Minuten | Ausbeute in % der Theorie | Selektivität in % der Theorie, bezogen auf umgesetzten Ausgangsstoff II |
|---|---|---|---|---|---|
| 13 | $BF_3$ | 0,1 | 20 | 45,9 | 96,0 |
| 14 | $AlCl_3$ | 0,2 | 5 | 47,7 | 92,4 |
| 15 | $FeCl_3$ | 0,2 | 5 | 45,4 | 93,9 |
| 16 | ®Lewatit SP 120 | 2 | 30 | 43,6 | 95,4 |
| 17 | $H_4SiW_{12}O_{40} \cdot 7H_2O$ | 0,3 | 60 | 44,8 | 94,3 |
| 18 | Montmorillonit (Typ KSF) | 0,3 | 400 | 43,7 | 93,6 |

*Tabelle 2*

| Beispiel | Alkohol | Zeit in Minuten | Trialkylorthoformiat Ausbeute in % der Theorie |
|---|---|---|---|
| 19 | $CH_3OH$ | 30 | 46 |
| 20 | $C_2H_5OH$ | 30 | 30 |
| 21 | n-$C_4H_9OH$ | 30 | 23 |

*Beispiele 22 bis 24*

Man verfuhr analog Beispiel 19, nur mit dem Unterschied, dass die in Tabelle 3 aufgeführten Ausgangsstoffe verwendet wurden.

*Tabelle 3*

| Beispiel | 1,3-Dioxolan | Alkohol | Zeit in Minuten | Trialkylorthoformiat Ausbeute in % der Theorie |
|---|---|---|---|---|
| 22 | 2-Methoxy- | $CH_3OH$ | 30 | 67 |
| 23 | 2-Methoxy-4-chlormethyl- | $CH_3OH$ | 30 | 56 |
| 24 | 2-Ethoxy-4-methyl- | $C_2H_5OH$ | 60 | 35 |

*Beispiel 25*

In einem 500-ml-Rührgefäss wurden 90 Gramm 2-Methoxy-4-methyl-(1,3)-dioxolan, 270 Gramm Methanol und 0,05 Gramm Trifluoressigsäure vorgelegt und bis zum Rückfluss erhitzt (65-70° C). Anschliessend tropfte man im Verlauf von 3 Stunden eine Lösung von 0,1 Gramm Trifluoressigsäure in 450 Gramm Methanol zu und destillierte ein Gemisch aus Endstoff und Methanol so ab, dass das Reaktionsvolumen konstant blieb. Anschliessend engte man das Gemisch bis zur Innentemperatur 90° C ein und versetzte Destillat und Rückstand mit je 2 Gramm $Na_2CO_3$.

Durch fraktionierte Destillation erhielt man aus dem Destillat 52 Gramm Trimethylorthoformiat vom Kp. 102° C (1010 mbar) und aus dem Rückstand 36 Gramm 1,2-Propandiol. Ausbeute: 67% (93%, bezogen auf umgesetztes 2-Methoxy-4-methyl-(1,3)-dioxolan) der Theorie.

*Beispiel 26*

a) In einem Rührgefäss legte man 1000 Gramm Methylformiat vor und versetzte mit 2 Gramm der Molekülverbindung $BF_3 \cdot HCOOCH_3$. Nach Spülen der Apparatur mit $N_2$ wurde innerhalb von 2 Stunden eine Mischung von 100 Gramm Propylenoxid und 100 Gramm Methylformiat unter Rühren zugegeben; die Reaktionstemperatur stieg dabei von 22° C auf 34° C an. Man rührte noch eine Stunde nach und destillierte das überschüssige Methylformiat ab.

b) Das Umsetzungsgemisch von 26a) wurde dann mit 550 Gramm Methanol versetzt und 30 Minuten auf 70° C erwärmt. Nach Neutralisation und fraktionierter Destillation analog Beispiel 25 erhielt man 71 Gramm Trimethylorthoformiat (39% der Theorie) vom Kp 102° C (1010 mbar), 87 Gramm unumgesetztes 2-Methoxy-4-methyl-(1,3)-dioxolan und 48 Gramm 1,2-Propandiol. Die Ausbeute bezieht sich auf eingesetztes Propylenoxid.

*Beispiel 27*

Ein Gemisch aus 1400 Gramm Methanol und 300 Gramm 2-Methoxy-4-methyl-(1,3)-dioxolan wurde durch eine auf 60° C erhitzte und mit 30 Gramm ®Lewatit SP 120 gefüllte Säule mit einer Verweilzeit von 30 Minuten geführt. Der Reaktionsaustrag wurde bei 400 mbar bis 60° C eingeengt. Das Destillat enthielt 145 Gramm (57% der Theorie) Trimethylorthoformiat vom Kp. 102° C (1010 mbar) und 35 Gramm unumgesetztes 2-Methoxy-4-methyl-(1,3)-dioxolan. Der Rückstand wurde in einer Extraktionskolonne mit n-Hexan extrahiert. Als untere Phase fallen 102 Gramm 1,2-Propandiol an. Aus der Hexanphase erhielt man nach Abziehen des Lösungsmittels weitere 62 Gramm 2-Methoxy-4-methyl-(1,3)-dioxolan (Umsatz: 67%).

*Beispiel 28*

In einem Siedegefäss, auf dem eine Glockenbodenkolonne mit 10 praktischen Böden aufgesetzt war, wurden 550 Gramm/Stunde Methanol verdampft. Am zweithöchsten Boden der auf 70 bis 75° C beheizten Kolonne wurden gleichzeitig 53 Gramm/Stunde einer Mischung aus 99,5 Gramm 2-Methoxy-4-methyl-(1,3)-dioxolan und 0,5 Gramm Trifluoressigsäure zudosiert. Das Kopfdestillat wurde bei einem Rücklaufverhältnis von 1 bis 2 entnommen und durch fraktionierte Destillation weiter getrennt. Nach einer Betriebsdauer von 6,5 Stunden erhielt man aus dem Destillat 283 Gramm Trimethylorthoformiat (92% der Theorie) und 27 Gramm 2-Methoxy-4-methyl-(1,3)-dioxolan. Der Sumpf wurde dreimal mit je 50 ml Hexan ausgeschüttelt, ebenfalls destilliert und lieferte 180 Gramm 1,2-Propandiol.

*Beispiel 29*

a) In einem Rührgefäss legte man 5250 Gramm Methylformiat vor und versetzte mit 10 Gramm der Molekülverbindung $BF_3 \cdot HCOOCH_3$. Nach Spülen der Apparatur mit $N_2$ wurden innerhalb von 2 Stunden 585 Gramm Propylenoxid unter Rühren zugegeben; die Reaktionstemperatur stieg dabei von 22° C auf 34° C an. Man rührte noch 20 Minuten nach und destillierte das überschüssige Methylformiat ab. Man erhielt 1138 g Destillationsrückstand (809 g 2-Methoxy-4-methyl-(1,3)-dioxolan).

b) In einem Siedegefäss, auf dem eine Glokkenbodenkolonne mit 20 praktischen Böden aufgesetzt war, wurden 630 g/Stunde Methanol verdampft. Am 16. (von unten gezählt) Boden der auf 70 bis 75° C beheizten Kolonne wurden gleichzeitig 138 Gramm/Stunde des in Beispiel 29a) erhaltenen Destillationsrückstandes zudosiert. Das Kopfdestillat wurde bei einem Rücklaufverhältnis von 1 bis 2 entnommen und durch fraktionierte Destillation weiter getrennt. Nach einer Betriebsdauer von 8,25 Stunden erhielt man aus dem Destillat 705 g Trimethylorthoformiat (97% der Theorie, bezogen auf verwendetes Dioxolan) vom Kp. 102° C (1010 mbar).

*Beispiel 30*

In einem Rührgefäss wurden 100 g 2,2,4-Trimethyl-(1,3)-dioxolan, 300 g Methanol und 0,5 g $BF_3$ (gelöst in 5 g zusätzlichem Methanol) während 2 Stunden bei 65° C gehalten. Über eine aufgesetzte Kolonne destillierte man dann bei 59 bis 61° C ein Gemisch aus Methanol (47 Gew.%) und 2,2-Dimethoxypropan (53 Gew.%) ab. Aus dem Gemisch wurde der Endstoff durch Azeotropdestillation mit Cyclohexan abgetrennt. Erhalten wurden 78 g (87% der Theorie) 2,2-Dimethoxypropan vom Kp. 83° C (1000 mbar).

*Beispiel 31*

In eine 2-m-Glockenbodenkolonne (20 Böden) wurden stündlich am 1. Boden von unten 171 g/h Methanol, am 8. Boden von unten 98 g/h 2,2-Dimethyl-(1,3)-dioxolan und am 2. Boden von oben 34 g/h der Katalysatorlösung (2,5 Gew.% $BF_3$ in Methanol) zudosiert. Bei einer Sumpftemperatur von 74° C fielen 182 g/h Destillat mit einem Siedepunkt von 59 bis 60° C an. Das Destillat enthielt 53,2 Gew.% 2,2-Dimethoxypropan und 46,8 Gew.% Methanol. Aus dem Gemisch wurde der Endstoff durch Azeotropdestillation mit Cyclohexan nach einer Reaktionsdauer von 5 Stunden abgetrennt. Man erhielt 484 g (97% der Theorie) 2,2-Dimethoxypropan vom Kp. 83° C (1000 mbar).

*Beispiel 32*

Man verfuhr analog Beispiel 31, verwendete jedoch als Zulauf 132 g/h Methanol, 125 g/h 2,2,4-Trimethyl-(1,3)-dioxolan und 40 g/h Katalysatorlösung. Man erhielt 197 g/h Destillat vom Siedepunkt 60° C, das 54 Gew.% 2,2-Dimethoxypropan enthielt. Aus dem Gemisch wurde der Endstoff durch Azeotropdestillation mit Cyclohexan nach einer Reaktionsdauer von 5 Stunden abgetrennt. Man erhielt 532 g (95% der Theorie) 2,2-Dimethoxypropan vom Kp. 83° C (1000 mbar).

## Patentanspruch

Verfahren zur Herstellung von aliphatischen Alkoxyverbindungen der Formel

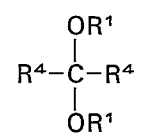

(I)

worin ein Rest $R^4$ ein Wasserstoffatom und der andere Rest $R^4$ die Gruppe $-OR^1$ bezeichnen oder beide Reste $R^4$ jeweils für einen Methylrest stehen, die einzelnen Reste $R^1$ gleich oder verschieden sein können und jeweils einen aliphatischen Rest mit 1 bis 6 Kohlenstoffatomen bedeuten, dadurch gekennzeichnet, dass man 2-substituierte (1,3)-Dioxolane der Formel

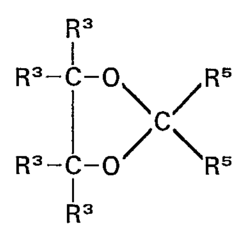

(II)

worin ein Rest $R^5$ ein Wasserstoffatom und der andere Rest $R^5$ den Rest

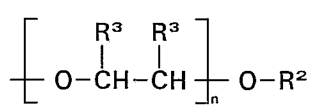

bedeuten oder beide Reste $R^5$ jeweils für einen Methylrest stehen, die einzelnen Reste $R^2$ und $R^3$ gleich oder verschieden sind und jeweils einen aliphatischen Rest bedeuten, $R^3$ auch für ein Wasserstoffatom, einen cycloaliphatischen, araliphatischen oder aromatischen Rest steht, und n eine ganze Zahl von 0 bis 3 bezeichnet, mit aliphatischen Alkoholen der Formel

$$R^1-OH \quad (III)$$

worin $R^1$ die vorgenannte Bedeutung besitzt, bei einer Temperatur von 0 bis 150° C

a) in Gegenwart eines Halogenids von Elementen der Gruppen IIb, IIIa, IVb, Va des Periodischen Systems und/oder von Eisen, Zinn, Silicium als Katalysatoren und gegebenenfalls einer Säure oder auch

b) wenn ein Rest $R^5$ ein Wasserstoffatom und der andere Rest $R^5$ den Rest

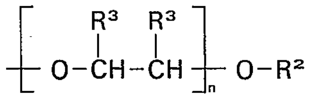

bedeuten, in Gegenwart einer Säure umsetzt.

## Claim

A process for the preparation of an aliphatic alkoxy compound of the formula

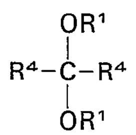

(I)

where one of the radicals $R^4$ is hydrogen and the other is $-OR^1$, or the two radicals $R^4$ are each methyl, and the individual radicals $R^1$ may be identical or different and are each an aliphatic radical of 1 to 6 carbon atoms, wherein a 2-substituted 1,3-dioxolane of the formula

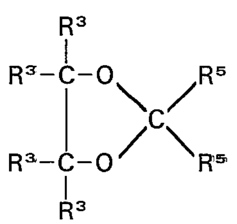

(II)

where one of the radicals $R^5$ is hydrogen and the other is

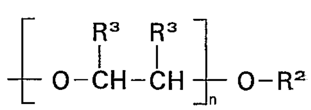

or the two radicals $R^5$ are each methyl, the individual radicals $R^2$ and $R^3$ are identical or different and are each an aliphatic radical, $R^3$ may furthermore be hydrogen or a cycloaliphatic, araliphatic or aromatic radical, and n is an integer from 0 to 3, is reacted with an aliphatic alcohol of the formula

$$R^1-OH \quad (III)$$

where $R^1$ has the above meanings, at from 0 to 150° C

(a) in the presence of a halide of an element of group IIb, IIIa, IVb or Va of the periodic table and/or of iron, tin or silicon as catalyst, and in the presence or absence of an acid, or

(b) when one of the radicals $R^5$ is hydrogen and the other is

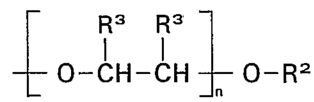

in the presence of an acid.

## Revendication

Procédé pour la préparation de composés alcoxylés aliphatiques de formule

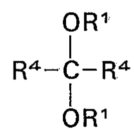

(I)

dans laquelle un radical $R^4$ désigne un atome d'hydrogène et l'autre radical $R^4$ le groupe $-OR^1$ ou les deux radicaux $R^4$ sont mis chacun pour un radical méthyle, les différents radicaux $R^1$ peuvent être semblables ou dissemblables et représentent chacun un radical aliphatique à 1-6 atomes de carbone, caractérisé en ce qu'on fait réagir des (1,3)-dioxolannes 2-substitués de formule

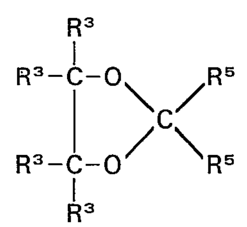

(II)

dans laquelle un radical $R^5$ représente un atome d'hydrogène et l'autre radical $R^5$ représente le radical

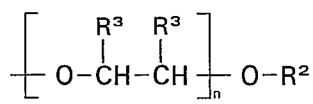

ou les deux radicaux $R^5$ sont mis chacun pour un radical méthyle, les différents radicaux $R^2$ et $R^3$ sont semblables ou dissemblables et représentent chacun un radical aliphatique, $R^3$ pouvant être également mis pour un atome d'hydrogène, un radical cycloaliphatique, araliphatique ou aromatique, et n est un nombre entier de 0 à 3, avec des alcools aliphatiques de formule

$$R^1-OH \qquad (III)$$

dans laquelle $R^1$ a la signification donnée ci-dessus, à une température de 0 à 150° C,

a) en présence d'un halogénure d'éléments des groupes IIb, IIIa, IVb, Va du système périodique et/ou de fer, d'étain, de silicium servant de catalyseur et, le cas échéant, d'un acide ou encore

b) dans le cas où un radical $R^5$ représente un atome d'hydrogène et l'autre radical $R^5$ représente le radical

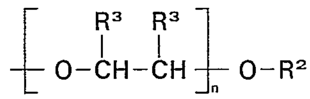

en présence d'un acide.